Europäisches Patentamt

(19) European Patent Office

Office européen des brevets

(11) EP 0 571 742 B1

(12) **EUROPÄISCHE PATENTSCHRIFT**

(45) Veröffentlichungstag und Bekanntmachung des Hinweises auf die Patenterteilung:
**02.01.1997 Patentblatt 1997/01**

(51) Int Cl.⁶: **C07C 227/40**, C12P 13/04, C12P 13/08

(21) Anmeldenummer: 93105321.9

(22) Anmeldetag: 31.03.1993

(54) **Verfahren zum Abtrennen von Aminosäuren aus wässrigen Lösungen**

Process for separating aminoacids from aqueous solutions

Procédé pour séparer des acides aminés de solutions aqueuses

(84) Benannte Vertragsstaaten:
**AT BE CH DE DK ES FR GB IE IT LI NL PT SE**

(30) Priorität: **23.05.1992 DE 4217203**

(43) Veröffentlichungstag der Anmeldung:
**01.12.1993 Patentblatt 1993/48**

(73) Patentinhaber: **Degussa Aktiengesellschaft**
**60311 Frankfurt (DE)**

(72) Erfinder:
- **Yonsel, Sems, Dr.**
  **W-6450 Hanau 1 (DE)**
- **Schäfer-Treffenfeldt, Wiltrud, Dr.**
  **W-6053 Obertshausen (DE)**
- **Kiss, Akos, Dr.**
  **W-6450 Hanau 9 (DE)**
- **Sextl, Elfriede, Dr.**
  **W-8752 Geiselbach (DE)**
- **Kinz-Naujok, Heike**
  **W-6450 Hanau 11 (DE)**

(56) Entgegenhaltungen:
EP-A- 0 299 434          FR-A- 2 562 067
FR-A- 2 587 337          FR-A- 2 666 996
US-A- 3 036 125          US-A- 3 565 951

- **THE JOURNAL OF PHYSICAL CHEMISTRY, Bd. 86, Nr. 9, 29. April 1982, Washington, DC, US, Seiten 1680 - 1683; T. IKEDA et al: "Kinetic behavior of L-lysine on zeolite X surfaces using the pressure-jump method"**

**Beschreibung**

Die Erfindung betrifft ein Verfahren zur Abtrennung von Aminosäuren aus wässrigen Lösungen.

Die technische Gewinnung einzelner Aminosäuren erfolgt über vier Wege:

Trennung der Aminosäuren aus natürlich vorkommenden und nachwachsenden Rohstoffen (z. B. Hühnerfedern, Schweineborsten);

Chemische Synthese (z. B. DL-Methionin);

Enzymatische Herstellung aus chemischen Vorstufen (z. B. L-Methionin)

Mikrobiologische Produktion, Fermentation (z. B. L-Lysin, L-Threonin, L-Tryptophan).

Bei all diesen Prozessen und Methoden stellt die Trennung und Isolierung der Aminosäuren einen der wesentlichen Verfahrensschritte dar.

Dazu werden häufig organische Ionenaustauscherharze eingesetzt.

L-Lysin wird z. B. an stark sauren Ionenaustauscherharzen des $NH_4^+$-Typs, bei einem pH-Wert von 0,5 bis 3 adsorbiert, der beladene Austauscher dann mit Ammoniakwasser eluiert und das gewünschte L-Lysinhydrochlorid durch Zugabe von Salzsäure gebildet (US -PS 3,565,951).

Tetsuya Ikeda et al. (J. phys. Chem. 86 (1982) 1680-1683) beschreiben das kinetische Verhalten bei der Adsorption von L-Lysin an Zeolithen des Typs X, wobei der entsprechende pH-Wert zwischen 9 und 10 liegt.

Untersuchungen des Desorptionsverhaltens finden sich hier nicht.

Aufgabe der Erfindung ist es, einen weiteren Weg zur Abtrennung aufzuzeigen, der es auch ermöglicht, das L-Lysinhydrochlorid oder -sulfat direkt zu gewinnen.

Gegenstand der Erfindung ist ein Verfahren zum Abtrennen von Aminosäuren aus wässrigen Lösungen, das dadurch gekennzeichnet ist, daß man diese Lösungen über Zeolithe leitet und die adsorbierten Aminosäure(n) anschließend durch Desorption aus den Zeolithen isoliert.

Die Lösungen stammen insbesondere aus Fermentationsprozessen zur Herstellung von Aminosäuren oder fallen bei Verfahren an, bei denen man durch die Hydrolyse von Naturprodukten, wie z. B. Hühnerfedern oder Schweineborsten, Aminosäure-haltige Lösungen gewinnt.

Ad- und Desorption werden bei Temperaturen zwischen 5 und 100 °C, insbesondere 15 bis 40 °C, durchgeführt.

Die Kinetik dieser Schritte wird durch die Temperatur nur in geringem Maße beeinflußt.

Im allgemeinen bewegt sich die Aminosäuren-Konzentration in den Lösungen bis zur Löslichkeitsgrenze, insbesondere 0,1 bis 15 Gew.-%.

Die Adsorption ist ebenso wie die Desorption pH-abhängig.

Dabei gilt, daß basische Aminosäuren, wie z. B. L-Lysin (pI=9,6), bevorzugt am isoelektrischen Punkt und im basischen Bereich adsorbiert werden (pH $\geq$ pI), während man neutrale Aminosäuren, z. B. L-Methionin oder L-Threonin (pI=5,7) und saure Aminosäuren, bevorzugt am isoelektrischen Punkt und im sauren Bereich (pH $\leq$ pI) adsorbieren läßt, und man anschließend die adsorbierte Aminosäure aus dem Zeolith nach dessen Abtrennung bei einem pH-Wert von <pI für basische Aminosäuren und einem pH-Wert >pI für neutrale und saure Aminosäuren eluiert.

Der pH-Wert, der sich beim Lösen einer Aminosäure in VE-(vollentsalztem) Wasser einstellt, entspricht in etwa auch dem isoelektrischen Punkt pI dieser Aminosäure.

Die zu behandelnden Lösungen werden daher bevorzugt durch Zusatz von Säuren bzw. alkalischen Substanzen auf diese pH-Bereiche eingestellt, in denen bevorzugt ad- bzw. desorbiert wird, wie es allgemein bekannt ist.

Dasselbe gilt, wenn sich während der Adsorption der pH-Wert der Lösung verschiebt.

Selbstverständlich müssen die Aminosäuren bei den jeweils zur Ad-bzw. Desorption eingestellten pH-Werten stabil sein.

Die Zeolithe können in Pulverform oder auch geformt z. B. in einem Festbett eingesetzt werden.

Als Formhilfsmittel dienen dann, wie allgemein bekannt, bevorzugt z. B. Alkylsilikate bzw. deren Hydrolysate oder Bentonite.

Für die Adsorption von Aminosäuren in wässrigen Lösungen werden Zeolithe mit unterschiedlichen Porengrößen, Strukturen, Dealuminierungsgraden und Kationen eingesetzt. Folgende Tabelle gibt eine Auswahl der Zeolithe mit ihren Eigenschaften an, die für den erfindungsgemäßen Zweck einsetzbar sind.

Tabelle 1

| Zeolithtypen für die Adsorption in Lösungen | | | |
|---|---|---|---|
| Zeolith | Modul $SiO_2$ /$Al_2O_3$ | Porenweite [Å] | Quelle |
| Zeolith A | ca. 2 | 3 - 5 | (1, 2) |

Tabelle 1   (fortgesetzt)

| Zeolithtypen für die Adsorption in Lösungen | | | |
|---|---|---|---|
| Zeolith | Modul SiO2 /Al2O3 | Porenweite [Å] | Quelle |
| Zeolith X | ca. 2 - 3 | ca. 7,4 | (1) |
| Zeolith Y, dealuminierter Zeolith Y (DAY) | 3 - ∞ | ca. 7,4 | (1, 3, 4) |
| Mordenit, dealuminierter Mordenit | ≥ 10 | 6,5 x 7 | (1, 5) |
| ZSM-5, dealuminierter ZSM-5 | 20 - ∞ | 5,3 x 5,6 | (5, 6) |
|  |  | 5,1 x 5,5 |  |
| Zeolith β | 20- ∞ | 7,5 x 5,7 | (7) |
|  |  | 6,5 x 5,6 |  |
| VIP-5 | - | 12,1 | (8) |

QUELLE 1 D.W. Breck, Zeolite Molecular Sieves, Structure, Chemistry and Use, J. Wiley & Sons, New York 1974;
2 H. Strack et al., (Degussa AG), DE 2660722 C2;
3 E. Roland et al., (Degussa AG), EP 0413138;
4 H.K. Beyer, I. Belenykaja, Catalysis by zeolites, Elsevier, Amsterdam 1980;
5 C.D. Chang (Mobil Oil Corp.), US-PS 4,273,753;
6 F.G. Dwyer et al., (Mobil Oil Corp.), DE 2836076 C2;
7 R.B. Calvert et al., (Mobil Oil Corp.), EP 0164208;
8 W.Schmidt et al., Zeolites 12 (1992), Jan., 2ff.

Es zeigt sich, daß die Beladung der verschiedenen Zeolithtypen von der Aminosäurekonzentration in der Lösung und von der Aminosäure selbst abhängt.

Daraus ergibt sich, daß für die Abtrennung jeder Aminosäure besonders geeignete Zeolithe existieren, die man durch Aufnahme der Adsorptionsisotherme leicht herausfinden kann.

So erreicht man bei einem pH Wert von 9 bis 10 L-Lysin an DAY eine maximal gemessene Beladung von etwa 12 bis 13 % an ZSM-5 oder Mordenit ~8 %, an NaY 10 %.

Für L- und DL-Methionin findet man bei pH 1 bis 6 die besten Beladungsergebnisse an ZSM-5 mit etwa 9 %. An DAY wird die Beladungsgrenze in dem untersuchten Konzentrationsbereich nicht erreicht; an Mordenit ist die maximale Beladung 4 %.

L-Threonin erreicht an DAY und ZSM die Beladung: 5 % an ZSM-5, 4 % an Mordenit und 1 % an DAY. Die Tendenz der Adsorbierbarkeit an gleichen Zeolithen ist bei L-Thr ähnlich wie bei L-/DL-Met, beide sind schwach saure Aminosäuren, wobei die Beladungen an ZSM-5 und DAY niedriger liegen.

Mit Hilfe des erfindungsgemäßen Verfahrens ist es jedoch nicht nur möglich, Aminosäuren aus wässrigen Lösungen zu entfernen.

Es gelingt außerdem, Aminosäuregemische zu trennen.

Mit Hilfe von ZSM-5 kann aus einer L-Lysin, L-Methionin und L-Threonin enthaltenden Lösung durch Adsorption im sauren pH-Bereich (pH ≃1) L-Methionin abgetrennt werden. Arbeitet man im alkalischen pH-Bereich (pH ≃9), wird L-Lysin aus diesem Gemisch selektiv adsorbiert.

Unter diesen Bedingungen kann man L-Lysin auch unter Verwendung von Zeolithen des Typs Mordenit oder DAY oder NaY abtrennen.

Die auf den jeweils eingesetzten Zeolithen adsorbierten Aminosäuren werden bei pH-Werten desorbiert, die für neutrale Aminosäuren (z. B. Methionin, Threonin), bevorzugt oberhalb, für basische Aminosäuren (z. B. Lysin), bevorzugt unterhalb des pI-Wertes liegen.

Auf diese Weise gelingt es, direkt z. B. Lysinhydrochlorid oder -sulfat zu erhalten.

Die Wiederfindungsrate des adsorbierten L-Lysins erreicht 100 % bei einem pH von ~1 an DAY und NaY. Als Säure wird für den Desorptionsschritt bevorzugt Schwefelsäure oder Salzsäure verwendet.

DL- und L-Methionin werden bei einem pH-Wert von ~10 vollständig desorbiert.

Das Verfahren kann entsprechend den Anforderungen kontinuierlich oder diskontinuierlich durchgeführt werden, beispielsweise indem man einen Seitenstrom aus einer Fermentation kontinuierlich durch ein Zeolithbett leitet und nach der Abtrennung der gewünschten Aminosäure wieder in den Fermentationsbehälter zurückführt.

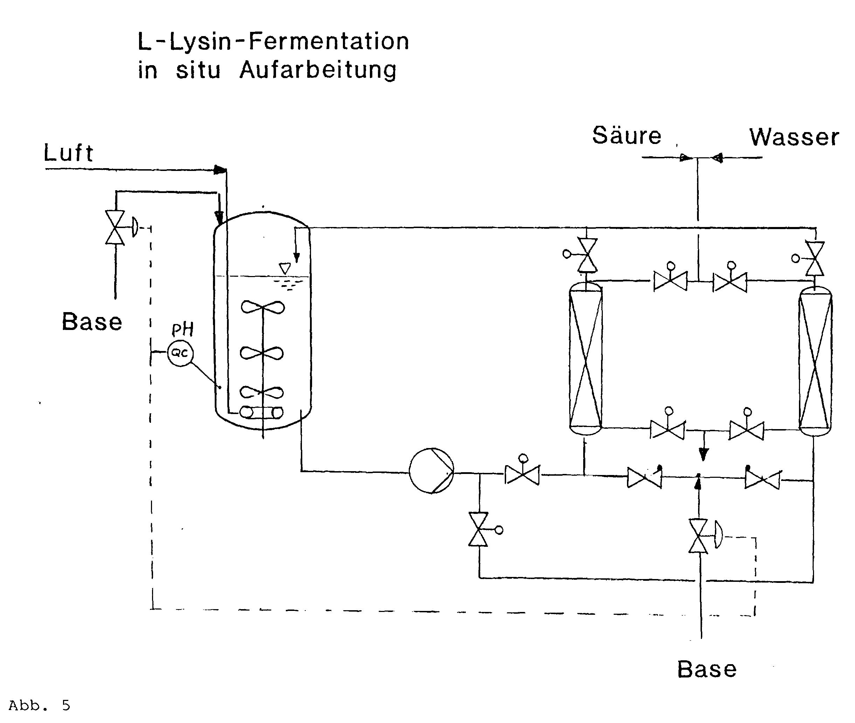

Abb. 5 zeigt ein Schema des Verfahrens. Eine Base z. B. Ammoniak wird während der Fermentation als pH-Korrekturmittel dem Reaktor zugegeben.

Zur in situ Aufarbeitung wird aus dem Reaktor im by pass über eine Sterilpumpe Fermentationsbrühe abgezogen und die Brühe über eine Säule mit der Zeolithpackung zurück zum Reaktor umgepumpt. Es findet keine Zellabtrennung

EP 0 571 742 B1

statt. L-Lysin wird an die Zeolithpackung adsorbiert während Lysin-arme Brühe in den Reaktor zurückkehrt.

Die Pumprate bzw. die Verweilzeit im by pass und Säule müssen so gewählt werden, daß die Zellen wegen Sauerstoff- und Substratmangel keinen Schaden erleiden.

Bei Bedarf schaltet man auch verschiedene Adsorptionsstufen hintereinander, die gegebenenfalls auch unter unterschiedlichen Bedingungen (pH-Wert, Zeolithtyp) betrieben werden, wenn z. B. verschiedene Aminosäuren voneinander getrennt werden sollen. Insgesamt ergibt sich eine Anzahl von Vorteilen, wenn man entgegen der nach dem Stand der Technik bekannten Verwendung von organischen Ionenaustauscherharzen Zeolithe zur Adsorption der Aminosäuren einsetzt.

Tabelle 2

Gegenüberstellung der Aufarbeitungsverfahren

Adsorption an Zeolithen und Ionenaustauschern

4

Adsorption

| an Zeolithen | an Ionenaustauscherharzen |
|---|---|
| -kein Regenerieren; Desorption ist zugleich Regenerationsschritt | -das Harz muß vor der Beladung mit Säure regeneriert werden |
| -dadurch geringere Salzfracht im Abwasser | |
| -Eluieren mit verschiedenen Säuren → (z.B. zur Herstellung verschiedener Lysin-Salze bei der Desorption | -nach dem Eluieren wird die Lösung mit der entsprechenden Säure zu den gewünschten Salzen umgewandelt (z.B. Lysin |
| -kein Aufquellen des Adsorbens | -das Aufquellen des Harzes verursacht Probleme: Verblockung, Kapazitätsverlust |
| -mechanische Festigkeit ist hoch, auch Bruchstücke der Formkörper erreichen maximale Beladungen | -zerbrochene Harzkugeln verlieren die Beladungseigenschaften |
| -erhöhte Temperaturen verringern die Adsorptionskapazität nicht hohe Temperaturbeständigkeit | |
| -Proteine, Biomasse, gelöste Salze stören die Adsorptionseigenschaften nicht | -Proteine beeinflussen, die Beladung negativ und müssen vorher abgetrennt werden; Fremdionen stören die Beladung. |

Beispiele

1. Aufarbeitung von Aminosäurelösungen

Die Adsorptionsexperimente von Aminosäuren an Zeolithen wurden statisch in gerührten bzw. geschüttelten 100 ml Kolben durchgeführt. Synthetische Aminosäure-Lösungen unterschiedlicher Konzentration ($C_0$ bis 80 g/l) wurden eingesetzt.

Folgende Zeolithtypen dienten als Adsorbentien

Tabelle 3

| | $SiO_2/Al_2O_3$ | Si/Al | Mikroporen-vol. (ml/g) | Kalzinierung (°C) (h) |
|---|---|---|---|---|
| NaY | 6 | 3 | 0,3 | keine |
| H-Mordenit | 20 | 10 | 0,2 | 550 1 |
| H-ZSM-5 | 45 | 23 | 0,2 | 550 1 |
| DAY | 200 | 100 | 0,3 | 950 1 |

Jeweils 3 g des pulverförmigen und mit Luftfeuchtigkeit gesättigten Zeoliths wurden eingewogen und z. B. einem Kolben mit 30 ml L-Lysin-Lösung zugegeben. Die Experimente liefen über Nacht (16 bis 20 h). Die Proben wurden filtriert und der Zeolith-freie Überstand mit Hilfe einer HPLC-Anlage analysiert.

Die Adsorptionsversuche wurden bei Raumtemperatur und bei 35°C und 60°C durchgeführt.

Die Bestimmung der adsorbierten Mengen erfolgten durch Analyse der Lysin-Konzentration am Anfang ($C_0$) und am Ende des Experiments ($C_f$). Der verbliebene Rest ist adsorbiert. Mit der Kenntnis der Adsorbenskonzentration ($C_z$ = g Zeolith/Aminosäure-Lösung) kann die Beladung X bestimmt werden:

$$X = \frac{C_0 - C_f}{C_z} \, [\%]$$

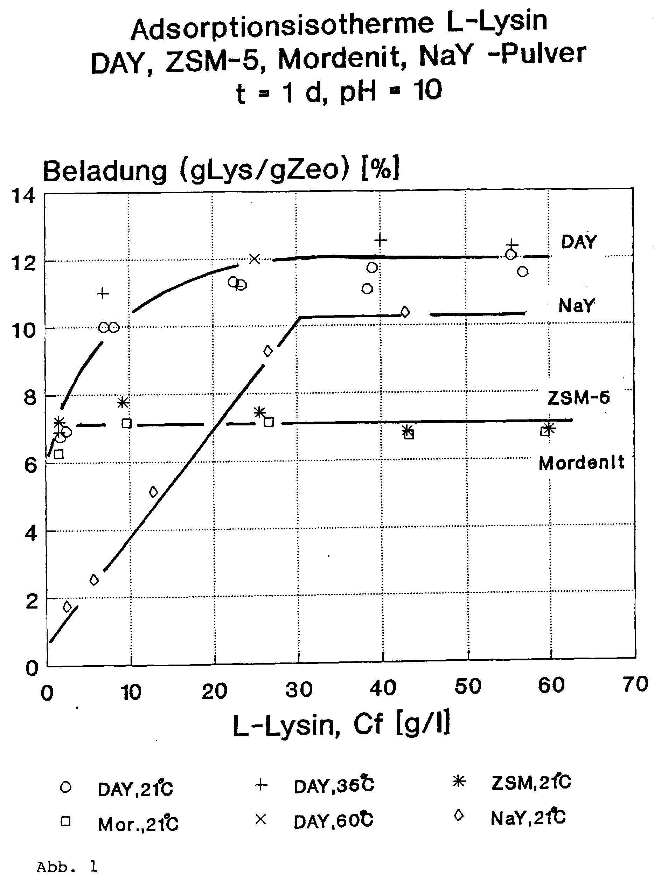

Abb. 1 zeigt die Adsorptionsisothermen von L-Lysin-Monohydrat. Die eingesetzten Lösungen hatten zusammen mit Zeolith einen durchschnittlichen pH-Wert von 9,5

Zwischen H-ZSM-5 und H-Mordenit sind keine Unterschiede feststellbar. An beiden Zeolithen wird eine maximale Beladung von etwa 8 % erreicht. Die Beladung von DAY unter gleichen Bedingungen (T = 21 °C) erreicht maximal etwa 13 % Experimente bei 35 °C und 60 °C zeigen, daß Temperaturerhöhungen keinen Einfluß auf die Adsorption haben,

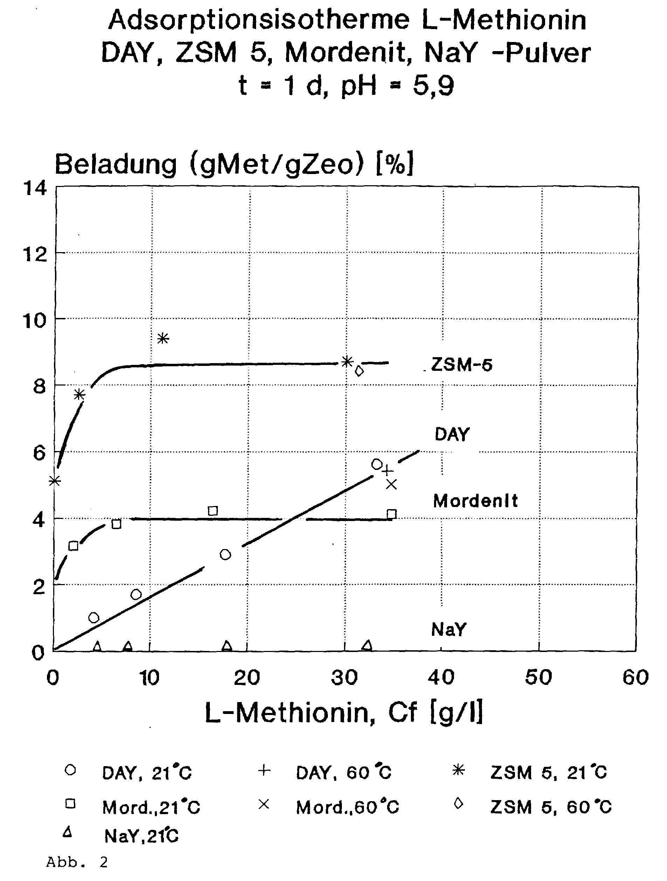

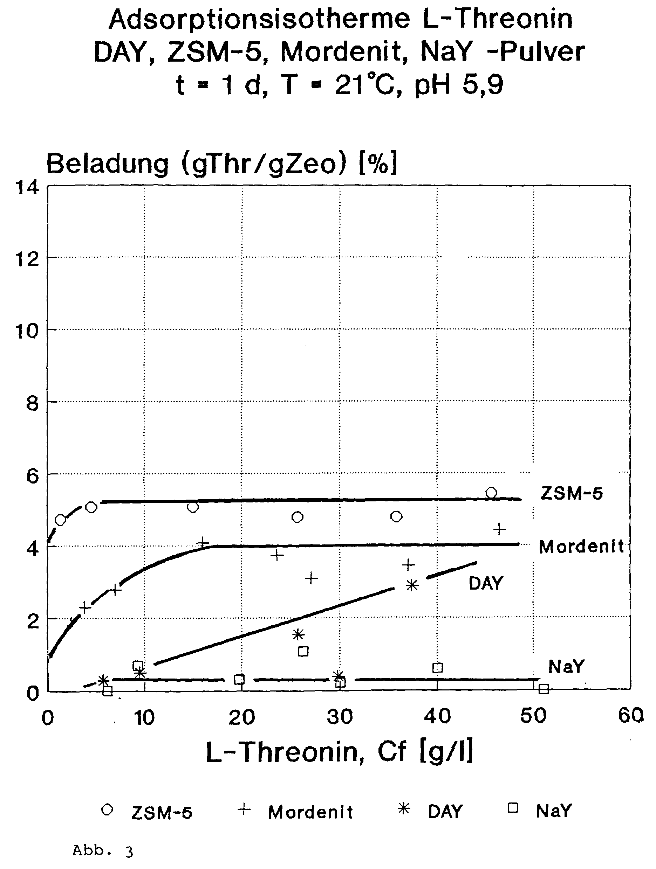

Analoge Adsorptionsversuche erfolgten mit DL- bzw. L-Methionin und L-Threonin.
Die Ergebnisse sind in den Abbildungen 2 bis 3 wiedergegeben.

2. Aufarbeitung von Fermentationsbrühen

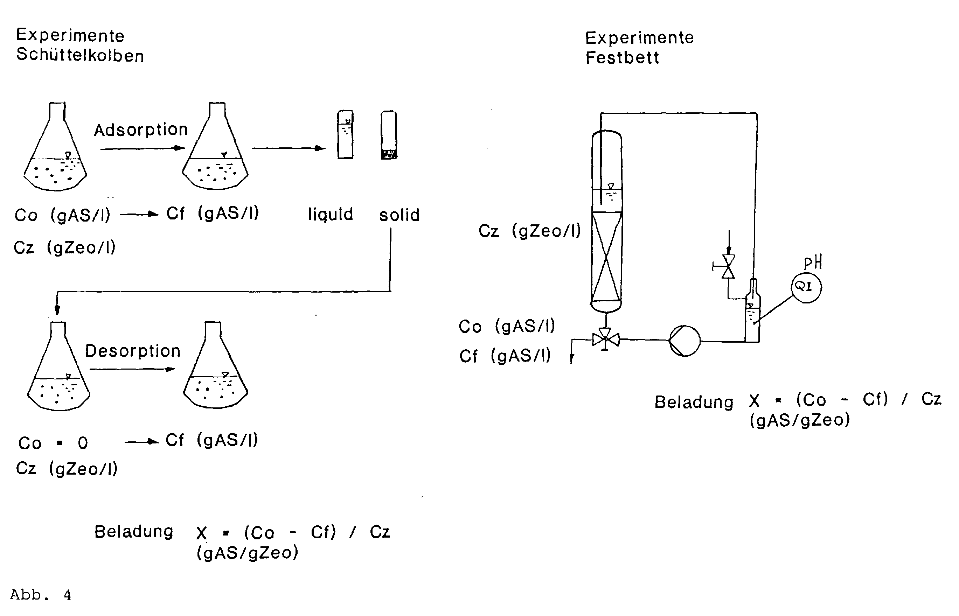

Die Ad- und Desorptionseigenschaften von L-Lysin wurden weiterhin in einer Fermentationsbrühe untersucht. Die Experimente wurden im Schüttelkolben mit Zeolith-Pulver und in der Festbettsäule mit Zeolith-Formkörpern bei verschiedenen pH-Werten durchgeführt (Abb. 4).

Aus einer laufenden Fermentation wurde eine Probe von etwa 4 l abgezogen und unmittelbar danach mit dem Antibiotikum Chloramphenicol (0,04 g/l) und Antimycotikum Pimaricin (0,01 g/l) versetzt und kaltgelagert. Damit stoppt man die mikrobielle Aktivität des Produktionsstammes und verhindert einen Fremdbefall. Da die Adsorptionsexperimente nicht unter sterilen Bedingungen durchgeführt werden, könnte eine Kontamination den Zerfall von Lysin verursachen und damit die Meßergebnisse im Laufe des Experiments verfälschen.

Das Medium enthält außer L-Lysin (74 g/l), das abzutrennen ist, mehrere andere Komponenten, komplexe Anteile, eine hohe Salzkonzentration, eine hohe Biomassen- bzw. Proteinkonzentration (Biotrockenmasse 30 g/l), mikrobielle Nebenprodukte sowie andere Aminosäuren. Der pH-Wert liegt bei 7,5.

Das Medium wurde ohne Abtrennung der Biomasse und ohne jegliche Vorbehandlung mit den Zeolithen in Kontakt gebracht. Im Schüttelkolben wurden pulverförmige Zeolithe und in der Festbettsäule DAY-Raschigringe (Durchmesser Außen 7 mm x Innen 4 mm) und H-ZSM-5-Vollzylinder (Durchmesser 3 mm) eingesetzt. Die Festbettsäulen aus Glas haben einen Innendurchmesser von 15 mm und eine Packungshöhe von 400 mm. Das Medium wurde in der Säule von unten nach oben gepumpt. Dabei wurde in der Pufferflasche der pH-Wert gemessen und nach Bedarf korrigiert. Als Korrekturmittel wurden Ammoniak und Schwefelsäure eingesetzt.

2.1. Ergebnisse

Der pH-Wert im Schüttelkolben wurde zwischen 7 und 10 variiert. Die Adsorptionskapazität (Beladung X) nimmt mit dem steigenden pH-Wert zu. Die maximalen Beladungen von 9 bis 12 % wurden an den Zeolithen DAY und NaY erreicht.

Ad- und Desorptionsexperimente sind mit der Fermentationsbrühe auch in der Festbettsäule mit DAY-Raschigringen bei pH-Werten zwischen pH 7,5 und 10 durchgeführt worden. Die Adsorptionskapazität von DAY erreicht bei dem Original-PH-Wert (pH 7,5) der Fermentationsbrühe eine Beladung von etwa 5 %. Mit steigendem pH-Wert kann die Kapazität auf $X = 12$ % (bei pH 10) erhöht werden.

Die Adsorptionskapazität von L-Lysin in der Fermentationsbrühe an DAY entspricht mit maximal 12% der Beladung in den synthetisch hergestellten Lösungen bei einem pH-Wert von etwa 10, was auf die hohe Selektivität trotz der vielen Fremdbestandteile in der Brühe hinweist.

Die DAY-Packung wurde mit einer Waschlösung (VE-Wasser und Salzsäure) bei pH 1 desorbiert. Die ursprüngliche Adsorptionsbeladung betrug 6 %. Es konnte eine Wiederfindungsrate ($g_{desorbiertes\ Lys}/g_{adsorbiertes\ Lys}$ in %) von nahezu 100 % erreicht werden. Die Ad-und Desorption wurden mehrmals bei veränderten pH-Werten bis zu 8 Zyklen in derselben Säule und mit derselben Packung wiederholt. Die DAY-Packung wurde dabei über eine Woche lang mit der Fermentationsbrühe beaufschlagt. Es wurden weder Verstopfungen noch ein Zuwachsen der Säule bzw. der Raschigringe beobachtet.

3. Aufarbeitung von Fermentationsbrühen in situ

Die bisher dargestellten Ergebnisse lassen die Möglichkeit zu, L-Lysin in situ während der Fermentation aufzuarbeiten. Abb. 5 zeigt ein Schema des Verfahrens. Die Base Ammoniak wird während der Fermentation als pH-Korrekturmittel dem Reaktor zugegeben.

Zur in situ Aufarbeitung wird aus dem Reaktor im by pass über eine Sterilpumpe Fermentationsbrühe abgezogen. Die Brühe wird über eine Säule mit der Zeolithpackung zurück zum Reaktor umgepumpt. Es findet keine Zellabtrennung statt. L-Lysin wird an die Zeolithpackung adsorbiert, Lysin-arme Brühe kehrt in den Reaktor zurück. Die Pumprate bzw. die Verweilzeit im by pass und Säule müssen so gewählt werden, daß die Zellen wegen Sauerstoff-und Substratmangels keinen Schaden erleiden.

Die Korrektur des pH-Wertes mit Ammoniak könnte statt an der Dosierstelle am Fermenter auch über die Adsorptionssäulen vorgenommen werden. (Abb. 5).

Dadurch entstehen zeitweilige ph-Gradienten, die die Adsorptionskapazität höher treiben. Dabei muß die Verweilzeit des Brühenstromes in der Säule sowie die Mischzeit und die Verteilung des pH-Wertes über die Säule so optimiert werden, daß die Mikroorganismen nicht beschädigt werden.

Die mit Lysin beladene Säule wird anschließend mit Wasser gewaschen und mit einer Säure eluiert. Durch Einsatz von Salz- oder Schwefelsäure kann das entsprechende Salz Lysin-Hydrochlorid oder Lysin-Sulfat auch alternierend hergestellt werden. Durch Einsatz von zwei Säulen kann während der Adsorption der ersten Säule die zweite desorbiert werden. Die folgenden Werte verdeutlichen die Vorteile des Verfahrens im Produktionsmaßstab.

| | |
|---|---|
| Reaktorvolumen | 300 m$^3$ |
| Arbeitsvolumen | 200 m$^3$ |
| Lysin-Konzentration | 70 g/l |
| kumuliertes Lysin | 14000 kg/Charge |
| Adsorptionskapazität DAY bei pH 7,5 | 5 % |
| Adsorptionskapazität DAY bei pH 9,5 | 10 % |
| Ad- bzw. Desorptionsdauer | 2 h |
| zwei Adsorptionssäulen jeweils | 25 m$^3$ |
| zwei Adsorptionssäulen mit jeweils | 10000 kg DAY-Raschigringe |
| in situ Aufarbeitungsphase | 40 h |
| Adsorption bei pH 7,5 alle 2 h | 500 kg Lysin |
| am Ende der Fermentation sind | 10000 kg Lysin aufgearbeitet |
| die restlichen 4000 kg Lysin werden nach der Produktion bei pH 9,5 in | 8 h getrennt |

Nach diesem Beispiel ist es möglich, 8 Stunden nach der Beendigung der Fermentation Lysin aus der Brühe vollständig zu trennen und zur Kristallisation in der gewünschten Form vorzubereiten.

**Patentansprüche**

1. Verfahren zum Abtrennen von Aminosäuren aus wässrigen Lösungen,

dadurch gekennzeichnet, daß man die Lösungen von basischen Aminosäuren (ioselektrischer Punkt pI > pH 7) auf einen pH-Wert ≥ pI, die von neutralen (pI zwischen pH 5 und 7) oder sauren (pI < pH5) Aminosäuren auf einen pH-Wert ≤ pI einstellt, sie dann mit einem geeigneten Zeolith in Kontakt bringt und die adsorbierte Aminosäure anschließend aus dem Zeolith nach dessen Abtrennung bei einem pH-Wert von < pI für basische Aminosäuren und einem pH-Wert > pI für neutrale und saure Aminosäuren eluiert.

2. Verfahren gemäß Anspruch 1,
dadurch gekennzeichnet, daß man die adsorbierten Aminosäuren mit der gewünschten Säure eluiert und das entsprechende Salz isoliert.

3. Verfahren gemäß den Ansprüchen 1 und 2,
dadurch gekennzeichnet, daß man Zeolithe der Typen A, X, Y, dealuminierter Y-Zeolith (DAY), Mordenit, dealuminierter Mordenit, ZSM-5, dealuminierter ZSM-5, Zeolith β oder VPI-5 einsetzt.

4. Verfahren gemäß den Ansprüchen 1 bis 3,
dadurch gekennzeichnet, daß man L-Lysin unter Verwendung der Zeolithtypen DAY, NaY, ZSM-5 oder Mordenit abtrennt.

5. Verfahren gemäß den Ansprüchen 1 bis 3,
dadurch gekennzeichnet, daß man DL-oder L-Methionin unter Verwendung der Zeolithtypen ZSM-5, DAY oder Mordenit abtrennt.

6. Verfahren gemäß den Ansprüchen 1 bis 3,
dadurch gekennzeichnet, daß man L-Threonin unter Verwendung der Zeolithtypen ZSM-5, Mordenit oder DAY abtrennt.

7. Verfahren gemäß einem oder mehreren der Ansprüche 1 bis 6,
dadurch gekennzeichnet, daß die wässrigen Lösungen Aminosäure-Gemische enthalten, und man die Aminosäuren getrennt isoliert.

8. Verfahren gemäß einem oder mehreren der Ansprüche 1 bis 7,
dadurch gekennzeichnet, daß man aus einer laufenden Fermentation in einem Seitenstrom Fermentationsbrühe abzieht, diese mit einem Zeolith in Kontakt bringt, an diesem adsorbiert und die an Aminosäure(n) abgereicherte Flüssigkeit wieder in den Fermentationsbehälter einspeist.

9. Verfahren gemäß Anspruch 7 und 8,
dadurch gekennzeichnet, daß man verschiedene Adsorptionsstufen zur Trennung der Aminosäuren hintereinander betreibt, in denen unterschiedliche Bedingungen eingehalten werden (pH-Wert, Zeolithtype).

10. Verfahren gemäß den Ansprüchen 8 und 9,
dadurch gekennzeichnet, daß man aus der Fermentationsbrühe die Biomasse nicht abtrennt.


**Claims**

1. Process for the separation of amino acids from aqueous solutions,
characterized in that the solutions of basic amino acids (isoelectric point pI > pH 7) are adjusted to a pH value ≥ pI, and those of neutral (pI between pH 5 and 7) or acidic (pI < pH 5) amino acids to a pH value ≤ pI, they are then contacted with a suitable zeolite, and the adsorbed amino acid is subsequently eluted from the zeolite after its separation at a pH value of < pI for basic amino acids and a pH value > pI for neutral and acidic amino acids.

2. Process according to Claim 1,
characterized in that the adsorbed amino acids are eluted with the desired acid and the corresponding salt is isolated.

3. Process according to Claims 1 and 2,
characterized in that zeolites of the types A, X, Y, dealuminized Y zeolite (DAY), mordenite, dealuminized mordenite, ZSM-5, dealuminized ZSM-5, zeolite-β or VPI-5 are used.

4. Process according to Claims 1 to 3,
characterized in that L-lysine is separated by use of the zeolite types DAY, NaY, ZSM-5 or mordenite.

5. Process according to Claims 1 to 3,
characterized in that DL- or L-methionine is separated by use of the zeolite types ZSM-5, DAY or mordenite.

6. Process according to Claims 1 to 3,
characterized in that L-threonine is separated by use of the zeolite types ZSM-5, mordenite or DAY.

7. Process according to one or more of Claims 1 to 6,
characterized in that the aqueous solutions contain mixtures of amino acids, and the amino acids are isolated separately.

8. Process according to one or more of Claims 1 to 7,
characterized in that
fermentation broth is withdrawn in a sidestream from a running fermentation and contacted with a zeolite, is adsorbed on the latter, and the liquid depleted in amino acid(s) is returned to the fermentation vessel.

9. Process according to Claims 7 and 8,
characterized in that,
for the separation of the amino acids, several adsorption steps are operated in series, wherein different conditions are maintained (pH value, zeolite types).

10. Process according to Claims 8 and 9,
characterized in that the biomass is not separated from the fermentation broth.


**Revendications**

1. Procédé pour séparer des acides aminés de solutions aqueuses,
caractérisé en ce qu'on règle les solutions d'acides aminés basiques (point isoélectrique pI > pH 7) à une valeur de pH $\geq$ pI, celles d'acides aminés neutres (pI entre pH 5 et 7) ou acides (pI < pH 5) à une valeur de pH $\leq$ pI, puis on les amène en contact avec une zéolithe appropriée et on élue ensuite l'acide aminé adsorbé de la zéolithe après séparation de cette dernière à une valeur de pH < pI pour des acides aminés basiques et à une valeur de pH > pI pour des acides aminés neutres et acides.

2. Procédé selon la revendication 1,
caractérisé en ce qu'on élue les acides aminés adsorbés avec l'acide désiré et on isole le sel correspondant.

3. Procédé selon les revendications 1 et 2,
caractérisé en ce qu'on met en oeuvre des zéolithes des types X, A, Y, de la zéolithe Y désaluminée (DAY), de la mordénite, de la mordénite désaluminée, ZSM-5, de la ZSM-5 désaluminée, de la zéolithe $\beta$ ou VPI-5.

4. Procédé selon les revendications 1 à 3,
caractérisé en ce qu'on sépare la L-lysine en utilisant les types de zéolithes DAY, NaY, ZSM-5 ou la mordénite.

5. Procédé selon les revendications 1 à 3,
caractérisé en ce qu'on sépare la DL- ou la L-méthionine en utilisant les types de zéolithes ZSM-5, DAY ou la mordénite.

6. Procédé selon les revendications 1 à 3,
caractérisé en ce qu'on sépare la L-thréonine en utilisant les types de zéolithes ZSM-5, la mordénite ou DAY.

7. Procédé selon une ou plusieurs des revendications 1 à 6,
caractérisé en ce que les solutions aqueuses contiennent des mélanges d'acides aminés et on isole séparément les acides aminés.

8. Procédé selon une ou plusieurs des revendications 1 à 7,

caractérisé en ce qu'on extrait un bouillon de fermentation issu d'une fermentation en continu dans un courant latéral, on l'amène en contact avec une zéolithe, on l'adsorbe sur cette dernière et on recycle le liquide appauvri en acide (s) aminé (s) dans le récipient de fermentation.

9. Procédé selon les revendications 7 et 8,
caractérisé en ce qu'on met en oeuvre successivement plusieurs étapes d'adsorption pour la séparation des acides aminés, dans lesquelles on maintient des conditions différentes (valeur de pH, type de zéolithe).

10. Procédé selon les revendications 9 et 10,
caractérisé en ce qu'on ne sépare pas la biomasse du bouillon de fermentation.

# Adsorptionsisotherme L-Lysin
## DAY, ZSM-5, Mordenit, NaY -Pulver
## t = 1 d, pH = 10

Abb. 1

# Adsorptionsisotherme L-Methionin
## DAY, ZSM 5, Mordenit, NaY -Pulver
## t = 1 d, pH = 5,9

Abb. 2

# Adsorptionsisotherme L-Threonin
## DAY, ZSM-5, Mordenit, NaY -Pulver
### t = 1 d, T = 21°C, pH 5,9

Beladung (gThr/gZeo) [%]

L-Threonin, Cf [g/l]

○ ZSM-5    + Mordenit    * DAY    □ NaY

Abb. 3

Experimente
Festbett

pH

QI

Cz (gZeo/l)

Co (gAS/l)
Cf (gAS/l)

Beladung $X = (Co - Cf) / Cz$
(gAS/gZeo)

Experimente
Schüttelkolben

Adsorption

liquid    solid

Co (gAS/l) $\longrightarrow$ Cf (gAS/l)
Cz (gZeo/l)

Desorption

Co = 0 $\longrightarrow$ Cf (gAS/l)
Cz (gZeo/l)

Beladung $X = (Co - Cf) / Cz$
(gAS/gZeo)

Abb. 4

14

L-Lysin-Fermentation
in situ Aufarbeitung

Abb. 5

EP 0 571 742 B1